# EUROPEAN PATENT SPECIFICATION

(11) **EP 3 821 907 B1**
(45) Date of publication and mention of the grant of the patent: **25.10.2023**
(21) Application number: 19923712.4
(22) Date of filing: 11.09.2019
(51) Int. Cl.: A61K 47/69, A61K 47/54, C12N 15/115, C07K 16/18, C07K 16/30, C07K 16/28, A61K 39/00, A61K 39/44, A61P 35/00

(54) **GOLD NANOPARTICLE-APTAMER CONJUGATE-BASED ANTIBODY DELIVERY SYSTEM, AND PREPARATION METHOD THEREOF**
AUF GOLDNANOPARTIKEL-APTAMER-KONJUGAT BASIERENDES ANTIKÖRPERFREISETZUNGSSYSTEM UND HERSTELLUNGSVERFAHREN DAFÜR
SYSTÈME D'ADMINISTRATION D'ANTICORPS BASÉ SUR UN CONJUGUÉ NANOPARTICULE D'OR-APTAMÈRE ET SON PROCÉDÉ DE PRÉPARATION

(30) Priority: 05.06.2019 KR 20190066491
(43) Date of publication of application: 19.05.2021
(73) Proprietor: NES BIOTECHNOLOGY CO., LTD., Seoul 06974 (KR)
(72) Inventor: LEE, Kang Seok, Gwangju-si, Gyeonggi-do 12813 (KR); BAE, Jee Hyeon, Gwangju-si, Gyeonggi-do 12813 (KR); YEOM, Ji-Hyun, Gwangju-si, Gyeonggi-do 12813 (KR); SHIN, Eunkyoung, Gwangju-si, Gyeonggi-do 12813 (KR); JOO, Min Ju, Gwangju-si, Gyeonggi-do 12813 (KR); HA, Hye Jeong, Gwangju-si, Gyeonggi-do 12813 (KR); LEE, Kang Seok, Gwangju-si, Gyeonggi-do 12813 (KR)
(74) Representative: Dehns
(86) International application number: PCT/KR2019/011838
(87) International publication number: WO 2020/246660

(56) References cited:
- WO-A2-2009/014404
- KR-A- 20130 012 773
- KR-A- 20130 041 591
- KR-A- 20140 102 596
- KR-A- 20180 002 645
- KR-A- 20180 064 585
- KR-B1- 101 613 020
- HA MINH HIEP ET AL: "RNA aptamer-based optical nanostructured sensor for highly sensitive and label-free detection of antigen-antibody reactions", ANALYTICAL AND BIOANALYTICAL CHEMISTRY, SPRINGER, BERLIN, DE, vol. 396, no. 7, 16 February 2010 (2010-02-16), pages 2575-2581, XP019798808, ISSN: 1618-2650

## Description

### [Technical Field]

The present invention relates to an antibody carrier based on a gold nanoparticle-aptamer conjugate and a method of preparing the same, and more particularly, to an antibody carrier which has the improved capability of delivering an antibody into cells by binding an immunoglobulin G (IgG)-specific aptamer with a gold nanoparticle and a method of preparing the same.

This application claims priority to and the benefit of Korean Patent Application No. 10-2019-0066491, filed on June 05, 2019.

### [Background Art]

Antibodies are biomolecules capable of most effectively recognizing and binding with a foreign substance, which have been retained in organisms over the past 4 billion years. Antibodies are Y-shaped immunoglobulin molecules specifically recognizing a target molecule, that is, an antigen, and binding with the antigen with high affinity, and play a pivotal role for vertebrate immune systems. Generally, antibodies are secreted from B lymphocytes or expressed on the surface of B lymphocytes, and have high individual diversity. Due to such diversity and specificity, antibodies can be used as neutralizers of pathogens or toxins, which are target substances in organisms, recruitment of immune components (complements, improvement in phagocytosis, cytotoxic antibodies derived from natural killer cells, etc.), various diagnosis procedures, or treatment for destroying a specific target.

Particularly, a monoclonal antibody (mAb) was approved for clinical use over the world to treat various diseases because of high specificity to an extracellular or intracellular antigen target. Since the first mAb approved in 1986, approximately 50 mAbs have been approved by the FDA. Antibody-based therapies used in medicine can target a protein of interest by disturbing protein-protein interactions or by inhibiting a signal transduction pathway. However, since most of these antibodies cannot be delivered into target cells, most of the antibodies approved by the FDA are antibodies targeting a receptor exposed on a cell surface. For example, trastuzumab (Herceptin/Herclon) targeting an HER2 receptor that is highly expressed and exposed on the surface is effectively used in treatment of a HER2-positive breast cancer patient.

A significant problem in which an antibody must be delivered into cells to be used as an effective therapeutic agent by being conjugated with a biomolecule in a target cell has to be overcome, and according to the development of nanobiotechnology, efforts have been made to deliver antibodies into cells using various nano carriers. However, antibodies have been delivered into cells using various substances such as a transmembrane peptide, hyaluronic acid, chitosan, dextran, a polyunsaturated fatty acid, a dendrimer, and a carbon nanotube, but these are delivered only into cells cultured *in vitro,* and there is no effective result for *in vivo* delivery. Moreover, the technologies developed so fat are only available for limited use by packaging and modification of specific antibodies.

Therefore, there is a pressing need to conduct research on a delivery system for easily and effectively delivering antibodies into a mammal without cytotoxicity.

Meanwhile, gold nanoparticles (AuNPs) are a type of novel nano substances developed as therapeutic agents and contrast agents for diagnosis, and currently have been widely used as a contrast agent for CT, MRI, positron emission tomology (PET), photo acoustic imaging (PA), or optical coherence tomology (OCT) in diagnostic radiology, and particularly, used as a contrast agent for CT. Since gold (Au) has a higher X-ray absorption rate and a higher molecular weight than a conventional iodine contrast agent, it is slowly released, thereby taking a longer imaging time, has high biocompatibility, thereby having low toxicity, and has a higher applicability as a contrast agent with a smaller amount than a conventional CT contrast agent. In addition, AuNPs have excellent *in vivo* stability, a unique characteristic of a large surface area that can be used in attachment of various molecules, and a potential to deliver biomolecules such as a peptide and a nucleic acid into cells and to be used as a cell therapeutic agent.

An aptamer, which is a short-length oligomer, has a characteristic of specifically binding to a target substance with high affinity by forming a stable three-dimensional structure, and has excellent advantages in terms of productivity because it can be mass-produced in a short time at low cost by using a chemical synthesis method and has almost no batch-to-batch variation. In addition, the aptamer is very highly stable to surrounding pH and temperature, and thus, recently, its applicability in various fields such as environmental and medical fields, for example, to detect a target substance and develop a disease diagnostic sensor, is highly evaluated.

Therefore, the inventors intended to develop an antibody carrier in which an aptamer binds to AuNP having excellent *in vivo* stability to effectively deliver an antibody for use in diagnosis or treatment of a disease.

WO2009/014404 discloses a polypeptide for intracellular delivery of an antibody and a nanoparticle, which comprises a nanoparticle-binding region, an antibody-binding region and a signalling molecule capable of delivering substances into cells.

KR101613020 discloses a gold nanoparticle bound to an aptamer specifically binding to the Fc region of an antibody for detecting a target antigen.

### [Disclosure]

### [Technical Problem]

Among immunoglobulins, immunoglobulin G (IgG) is most abundant, and the inventors had conducted research on aptamers for IgG to be applied and used in potential applications for analysis, development of a new drug and affinity purification, resulting in development of a novel delivery system for delivering antibodies into cells by binding a DNA aptamer capable of binding to a common region of mouse IgG-Fc domains and a DNA aptamer capable of binding to a fluorescent material, which is fluorescein isothiocyanate (FITC), and gold nanoparticles (AuNPs). Thus, the present invention was completed.

Therefore, the present invention is directed to providing an antibody carrier, which comprises AuNP; and an aptamer binding to the surface of the AuNP.

The present invention is also directed to providing a method of preparing an antibody carrier, which comprises binding an aptamer with AuNP.

However, technical problems to be solved in the present invention are not limited to the above-described problems, and other problems which are not described herein will be fully understood by those of ordinary skill in the art from the following descriptions.

### [Technical Solution]

To attain the above-described objects, the present invention provides an antibody carrier, which comprises AuNP; and an aptamer binding to the surface of the AuNP as defined in the claims.

In addition, the present invention provides a method of preparing an antibody carrier, which comprises binding said aptamer to AuNP.

The aptamer may be an aptamer specifically binding to the Fc domain of immunoglobulin G (IgG) or the fluorescent material, fluorescein isothiocyanate (FITC).

The aptamer specifically binding to the Fc domain of the IgG consists of a base sequence of SEQ ID NO: 1.

In still another embodiment of the present invention, the aptamer specifically binding to the FITC consists of a base sequence of SEQ ID NO: 2.

In yet another embodiment of the present invention, the antibody carrier may be prepared by binding one or more monoclonal antibodies selected from the group consisting of IgG, PARP1, VP6, NSD2, Aurora A, Pax5, Vimentin, Rock-1, Rock-2, RhoE, P53, Mcl-1, P50 and BRAF to the aptamer and delivered into cells.

In yet another embodiment of the present invention, the AuNP may have a diameter of 10 to 20 nm.

In yet another embodiment of the present invention, the antibody carrier may deliver antibodies into one or more selected from the group consisting of the cell nucleus, the cytoplasm and mitochondria.

In addition, the present invention provides a complex formed by binding the antibody carrier with antibodies to be delivered.

In addition, the present invention provides a pharmaceutical composition for use in preventing or treating cancer, which comprises a complex formed by binding the antibody carrier with antibodies to be delivered, as an active ingredient.

In addition, the present invention provides a pharmaceutical composition for use in a method of preventing or treating cancer, which comprises administering a pharmaceutical composition comprising a complex formed by binding the antibody carrier with antibodies to be delivered into a subject.

### [Advantageous Effects]

An antibody carrier according to the present invention is prepared by binding of an immunoglobulin G (IgG) Fc domain-specific aptamer or a fluorescein isothiocyanate (FITC)-specific aptamer to a gold nanoparticle (AuNP), and can effectively deliver an antibody into the cell nucleus, the cytoplasm and mitochondria by specific binding of the antibody to be delivered to the aptamer. In addition, since the AuNP having very low cytotoxicity is used, the antibody carrier is not only harmless to the human body, but can also detect a location in cells easily by reflecting light at various wavelengths, and therefore, it is expected to be effectively used in diagnosis or treatment of a disease.

### [Description of Drawings]

FIG. 1 is a schematic diagram illustrating a process of preparing an aptamer-gold nanoparticle (AuNP)-antibody complex by binding an immunoglobulin G (IgG)-Fc domain-specifically binding DNA aptamer and an antibody to AuNP according to an embodiment of the present invention.
FIG. 2A is a set of confocal microscope images showing the capability of delivering antibodies into HeLa cells by AuNP-Apt^{IgG} according to an embodiment of the present invention.
FIG. 2B is a set of confocal microscope images showing the capability of delivering antibodies into A549 cells by AuNP-Apt^{IgG} according to an embodiment of the present invention.
FIG. 3 is a set of confocal microscope images showing the capability of delivering antibodies into Malme-3M cells and SK-Mel2 cells by AuNP-Apt^{FITC} according to an embodiment of the present invention.
FIG. 4 shows results of measuring cell viability after A2058, Malme-3M and SK-MEL2 cells are treated with AuNP-Apt^{IgG}-BRAF^{V600E} at various concentrations (0, 0.88, 1.65 and 3.3 nM) and incubated according to one embodiment of the present invention.

### [Modes of the Invention]

The present invention provides an antibody carrier, which comprises a gold nanoparticle (AuNP); and
an aptamer binding to the surface of the AuNP.

The "nanoparticle" used herein includes particles of various substances having a nano-sized diameter, and the nanoparticle is not particularly limited as long as it is a nano-sized particle.

The "gold nanoparticle" used herein refers to a metal particle of gold having a nano-sized diameter, and such a small particle size allows the nanoparticle of the present invention to penetrate into target cells (e.g., human cells), and allows penetration of a protein carrier into cells.

AuNPs are easily prepared in the form of stable particles, and may vary in size from 0.8 to 200 nm according to the purpose of use. In addition, gold may be bound with various types of molecules, such as a peptide, a protein, and a nucleic acid, thereby changing its structure, and reflect light at various wavelengths, thereby easily confirming a location in a cell. Moreover, AuNPs are not harmful to the human body, unlike heavy metals such as manganese, aluminum, cadmium, lead, mercury, cobalt, nickel, and beryllium so that they have high biocompatibility and have very low cytotoxicity. In addition, when AuNPs have a diameter of 100 nm or more, characteristics as a nanoparticle are lost, and the binding between the gold surface not having the characteristics of a nano substance and a functional group such as a thiol group is weaker. Since AuNPs having a diameter outside 10 to 20 nm induce cytotoxicity, in the present invention, the AuNP preferably has a diameter of 10 to 20 nm, but the present invention is not limited thereto.

The "aptamer" used herein refers to an oligonucleotide substance, which is a single-stranded nucleic acid (DNA, RNA or modified nucleic acid) having a stable three-dimensional structure as it is and able to bind to a target molecule with high affinity and specificity, enables chemical synthesis and has comparatively flexible features with respect to heat and pH changes. By a method called Systematic Evolution of Ligands of Exponential enrichment (SELEX), aptamers for various target substances of interest (a protein, a peptide, a lipid, an inorganic compound, a low-molecular organic substance, a saccharide, a staining substance, DNA, a metal ion, a cell, an antibiotic, etc.) may be developed.

In the present invention, the aptamer may be an aptamer specifically binding to the Fc domain of immunoglobulin G (IgG), which consists of a base sequence of SEQ ID NO: 1, or an aptamer specifically binding to fluorescein isothiocyanate (FITC), which is a fluorescent material attached to label a delivered antibody, which consists of a base sequence of SEQ ID NO: 2. Here, both of the sequences of the aptamers may have modifications at the 3' end with a thiol group.

The antibody carrier according to the present invention may effectively deliver antibodies into cells by specific binding between an IgG Fc domain-specific aptamer binding to AuNP and the Fc domain of an antibody to be delivered, and here, the antibody carrier may deliver antibodies into cells by binding of one or more monoclonal antibodies selected from the group consisting of IgG, PARP1, VP6, NSD2, Aurora A, Pax5, Vimentin, Rock-1, Rock-2, RhoE, P53, Mcl-1, P50 and BRAF to the aptamer, but the antibody carrier is not limited to the type of antibody.

In addition, the antibody carrier according to the present invention may deliver antibodies into cells by specific binding of an AuNP-binding FITC-specific aptamer to FITC, which is a fluorescent material for labeling an antibody to be delivered. Here, there is no particular limitation on an antibody type when an antibody is labeled with FITC, but according to an exemplary embodiment of the present invention, the antibody carrier may specifically bind to FITC used to label IgG, thereby delivering IgG into cells.

In the present invention, the antibody carrier may deliver antibodies into cells, and for example, deliver antibodies into one or more selected from the group consisting of the cell nucleus, the cytoplasm and mitochondria.

In addition, the present invention provides a method of preparing an antibody carrier, which comprises binding an aptamer to AuNP.

Here, the antibody carrier may be prepared by binding an aptamer specifically binding to the Fc domain of IgG to AuNP or binding an aptamer specifically binding to FITC to AuNP.

In addition, the present invention provides a complex formed by binding the antibody carrier with an antibody to be delivered.

In addition, the present invention provides a pharmaceutical composition for use in preventing or treating cancer, which comprises a complex formed by binding the antibody carrier with an antibody to be delivered as an active ingredient.

The term "prevention" used herein refers to all actions of inhibiting or delaying cancer by administration of the composition according to the present invention.

The term "treatment" used herein refers to all actions involved in alleviating or beneficially changing symptoms of cancer by administration of the composition according to the present invention.

The term "pharmaceutical composition" used herein is prepared for the purpose of preventing or treating cancer, and may be formulated into various forms according to a conventional method. For example, the pharmaceutical composition may be formulated into oral preparations such as powder, a granule, a tablet, a capsule, a suspension, an emulsion or a syrup, and may be formulated into a skin preparation for external use such as a cream, a gel, a patch, a spray, an ointment, a plaster, a lotion, a liniment, a pasta or a cataplasma, a suppository or a sterile injectable solution.

The pharmaceutical composition according to the present invention may further include a carrier, an excipient and a diluent, which are suitably and commonly used in the preparation of pharmaceutical compositions. Here, the carrier, excipient and diluent, which can be used in the composition, may include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil. In preparation, the composition may be formulated with a diluent or an excipient such as a filler, a thickening agent, a binder, a wetting agent, a disintegrant, a surfactant, which are conventionally used. A solid formulation for oral administration may be a tablet, pill, powder, granule or capsule, and such a solid formulation may be prepared by mixing at least one of excipients, for example, starch, calcium carbonate, sucrose, lactose and gelatin, with the active ingredient. Also, in addition to the simple excipient, lubricants such as magnesium stearate and talc may also be used. As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration includes a sterilized aqueous solution, a non-aqueous solvent, a suspension, an emulsion, a lyophilizing agent and a suppository. As the non-aqueous solvent or suspension, propylene glycol, polyethylene glycol, vegetable oil such as olive oil, or an injectable ester such as ethyl oleate may be used. As a suppository base, Witepsol, Macrogol, Tween 61, cacao butter, laurin butter, or glycerogelatin may be used.

The pharmaceutical composition of the present invention may be administered orally or parenterally (e.g., intravenously, subcutaneously, intraperitoneally or locally) according to a desired method, and a dose may be suitably selected by those of ordinary skill in the art according to a patient's condition and body weight, the severity of a disease, a dosage form, an administration route and duration.

The pharmaceutical composition of the present invention is administered at a pharmaceutically effective amount. The "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field. The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by those of ordinary skill in the art. The dose may be administered once or in divided portions a day.

The "cancer" used herein is the generic term for diseases caused by cells having an aggressive characteristic in which cells divide and grow regardless of a normal growth limit, an invasive characteristic in which cells penetrate into surrounding tissue, and a metastatic characteristic in which cells spread to a different site in the body. In the present invention, the cancer may be one or more selected from the group consisting of liver cancer, breast cancer, blood cancer, prostate cancer, ovarian cancer, pancreatic cancer, stomach cancer, colon cancer, brain cancer, thyroid cancer, bladder cancer, esophageal cancer, cervical cancer, skin cancer and lung cancer, but the present invention is not limited thereto.

In addition, the present invention provides a pharmaceutical composition for use in a method of preventing or treating cancer, which comprises administering the pharmaceutical composition comprising a complex formed by binding the antibody carrier with an antibody to be delivered into a subject.

In addition, the present invention provides a method of delivering an antibody, which comprises administering the complex into a subject.

The term "subject" used herein refers to a target in need of diagnosis or treatment of a disease, and more specifically, a mammal such as a human or a non-human primate, a mouse, a rat, a dog, a cat, a horse, or a cow.

The term "administration" used herein refers to providing the complex of the present invention to a subject by a suitable method.

In one embodiment of the present invention, an antibody carrier (AuNP-Apt^{IgG}) in which an IgG Fc domain-specific aptamer is conjugated with AuNP and an antibody carrier (AuNP-Apt^{FITC}) in which an FITC-specific aptamer is conjugated with AuNP are prepared (refer to Example 2), and as a result of confirming the capability of delivering an antibody into cells by the AuNP-Apt^{IgG} (refer to Example 3) and the capability of delivering an antibody into cells by the AuNP-Apt^{FITC} (refer to Example 4), it was confirmed that antibodies were effectively delivered into cells by the antibody carriers AuNP-Apt^{IgG} and AuNP-Apt^{FITC}.

In another experimental example of the present invention, as a result of delivering a BRAF^{V600E} antibody into a SK-MEL2 cell line and BRAF mutant (BRAF^{V600E}) cell lines A2058 and Malme-3M using an AuNP-Apt^{IgG}-BRAF^{V600E} complex, a decrease in cell viability in the human skin cancer cell lines A2058 and Malme-3M was confirmed, demonstrating that an AuNP-aptamer-antibody complex can be used as a cell therapeutic agent (refer to Example 5).

Hereinafter, to help in understanding the present invention, exemplary examples will be suggested. However, the following examples are merely provided to more easily understand the present invention, and not to limit the present invention.

### Example 1. Experimental method

### 1-1. Mammalian cell culture

Human cervical carcinoma (HeLa) cells were cultured in Dulbecco's modified Eagle's medium (DMEM), and human epidermoid carcinoma (A549) cells were cultured in an RPMI-1640 medium containing a 2.5 mM N-(2-hydroxyethyl)piperazine-N'-2-ethanesulfonate (HEPES) buffer. In addition, Malme-3M and SK-MEL-2 cells, which are human melanoma cells, were cultured in an RPMI-1640 medium, and A2058 cells were cultured in a Dulbecco's modified Eagle's medium. All media contain 10% heat-inactivated fetal bovine serum (Caisson, USA) and 1% penicillin-streptomycin (Welgene, Korea).

### 1-2. Preparation of AuNP-aptamer-antibody complex

To prevent the formation of a secondary structure, an AuNP-aptamer conjugate was pre-incubated for 5 minutes at 80 °C. In addition, the AuNP-aptamer conjugate (AuNP-Apt^{IgG}) (1 nM) and a monoclonal antibody were reacted in 1x PBS containing 5 mM MgCh (pH 7.2) for 10 minutes at room temperature, and then a supernatant was removed by centrifugation at 13,000× g. The AuNP-Apt^{IgG}-antibody complex prepared thereby was washed using TBST supplemented with 500 mM NaCl and 1M KCI.

### 1-3. Analysis of antibody delivery capability by AuNP-aptamer conjugate using confocal microscope

To observe antibody delivery into cells, cells cultured on a lysine-coated 10-mm cover slip were treated with an AuNP-aptamer-antibody complex (AuNP-Apt^{IgG} - antibody complex), incubated for 1 hour, and the fixed with 4% paraformaldehyde (Sigma, USA). Afterward, FITC (490 nm excitation, 525 nm emission) and Alexa 546 (556 nm excitation, 573 nm emission) fluorescence was detected by laser scanning confocal microscopy (Carl Zeiss ZEN 2011, Germany).

### 1-4. Cell viability assay

A cell line was seeded in a 96-well plate, and incubated overnight such that the cells were attached thereon. Afterward, the cells were incubated with AuNP-Apt^{IgG}-BRAF^{V600E} at different concentrations (0, 0.88, 1.65 and 3.3 nM), and cell viability was measured (Yeom, J.H. et al. Inhibition of Xenograft Tumor Growth by Gold Nanoparticle-DNA Oligonucleotide Conjugates-Assisted Delivery of BAX mRNA. PLoS One 8, e75369 (2013)).

### Example 2. Preparation of antibody carrier using AuNP-aptamer conjugate

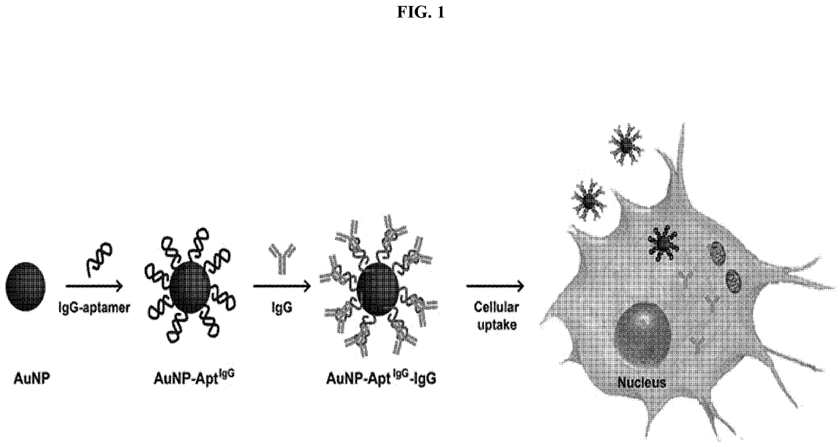

An antibody carrier was prepared using an aptamer binding to the Fc domain of a monoclonal antibody, and the schematic diagram thereof is shown in FIG. 1.

### 2-1. Preparation of antibody carrier (AuNP-Apt^{IgG}) using IgG aptamer

### A) Pretreatment of DNA aptamer

To prepare an antibody carrier according to the present invention, an IgG-aptamer detecting the Fc domain of a monoclonal antibody and specifically binding thereto was used. More specifically, the IgG-aptamer is an aptamer in which the 3' end is modified with a thiol group, and consists of a base sequence of 5'-TAATACGACTCACTATAGCAATGGTACGGTACTTCCCCACTCACCGGGTAC CTGCCGCTCCCAAAAGTGCACGCTACTTTGCTAAAAAAAAAAAA-3'(SH) (SEQ ID NO: 1). The dried DNA aptamer was dissolved in water to have a final concentration of 100 µM, and 10 µL of 1N dithiothreitol (DTT) was added to 50 µL of oligo and reacted for 15 minutes at room temperature. To remove DTT containing an unwanted thiol molecule, 50 µL of ethyl acetate was added and stirred, and then the resulting product was centrifuged to remove a supernatant. This process was repeated three times. In addition, the aptamer was precipitated by an EtOH precipitation method.

### B) Binding of AuNP with DNA aptamer

The IgG-aptamer precipitated by pretreatment through the A) step was dissolved in water, and then added to AuNP. Here, as the AuNP, Gold colloid-15 nm (#EM.GC15) purchased from BBI Life Science (UK) was used.

Specifically, an aptamer was added to 2 nM AuNP (DNA: AuNP = 100:1) and sufficiently stirred, and then a 0.5 M citrate buffer (pH 3) was added and mixed with the resulting mixture to have the final concentration of 10 mM. After a reaction for 3 to 5 minutes at room temperature, for gold neutralization, a 0.5 M HEPES buffer (pH 7.6) was added to have the final concentration of 30 mM and reacted for 10 minutes at room temperature. The mixture of the aptamer and gold was centrifuged for 20 minutes at ~10,000x g and concentrated, and then an unreacted oligo in the supernatant was removed. This process was repeated three times. The final AuNP-aptamer conjugate was dispersed in a 5 mM HEPES buffer (pH 7.6).

### 2-2. Preparation of antibody carrier (AuNP-Apt^{FITC}) using FITC aptamer

### A) Pretreatment of DNA aptamer

To prepare an antibody carrier according to the present invention, an fluorescein isothiocyanate (FITC)-aptamer (FITC-aptamer) specifically binding to FITC widely used as a fluorescent tracer was used. More specifically, the FITC-aptamer is an aptamer in which the 3' end is modified with a thiol group, and consists of a base sequence of 5'-GGACGGCACCACGGTCGGATCCGTGAGTTGTGACAATTTAGCGGGTGGTA TTAGAGCCTACTGCCACAGCAATAGGATCGATACAGATCTAAAAAAAAAA-3' (SH) (SEQ ID NO: 2). The dried DNA aptamer was dissolved in water to have the final concentration of 100 µM, and 10 µL of 1N DTT was added to 50 µL of oligo and reacted for 15 minutes at room temperature. To remove DTT containing an unwanted thiol molecule, 50 µL of ethyl acetate was added and stirred, and then the resulting product was centrifuged to remove a supernatant. This process was repeated three times. Afterward, the aptamer was precipitated using an EtOH precipitation method.

### B) Binding between AuNP and DNA aptamer

An antibody carrier (AuNP-Apt^{FITC}) was prepared by binding the FITC-aptamer precipitated by pretreatment through the A) step to AuNP by the same method as described in B) step of Example 2-1.

### Example 3. Confirmation of capability of delivering antibody into cells by AuNP-Apt^{IgG}

### 3-1. Confocal microscopy

An AuNP-aptamer-antibody complex was prepared using the AuNP-aptamer conjugate prepared according to Example 2-1 by the method described in Example 1-2. Subsequently, HeLa cells and A549 cells were treated with the AuNP-aptamer-antibody complex, and then incubated for 1 hour. After incubation, the cells were washed with PBS, fixed with 4% paraformaldehyde, and the fixed sample was immunostained and observed using a confocal microscope.

### 3-2. Confirmation of capability of delivering antibody into cells by AuNP-AptIgG

AuNP-aptamer-antibody complexes were prepared by reacting monoclonal antibodies PARP1, VP6, NSD2, Aurora A, Pax5, Vimentin, Rock-1, Rock-2, RhoE, P53, Mcl-1, P50 and BRAF with AuNP-Apt^{IgG}. Afterward, the complexes were labeled with a secondary antibody Mouse-546 (Red) and observed by confocal microscopy as described in Example 3-1 to confirm whether the antibodies were delivered into the HeLa and A549 cells.

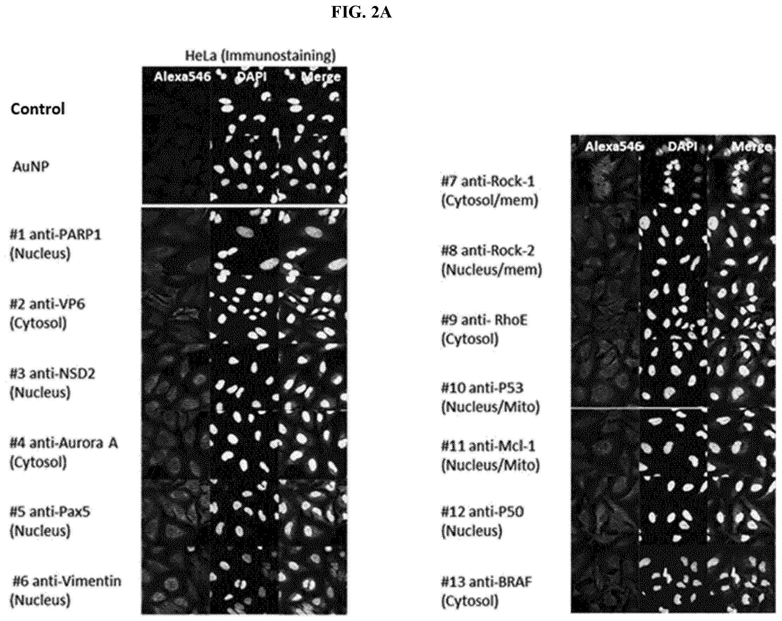

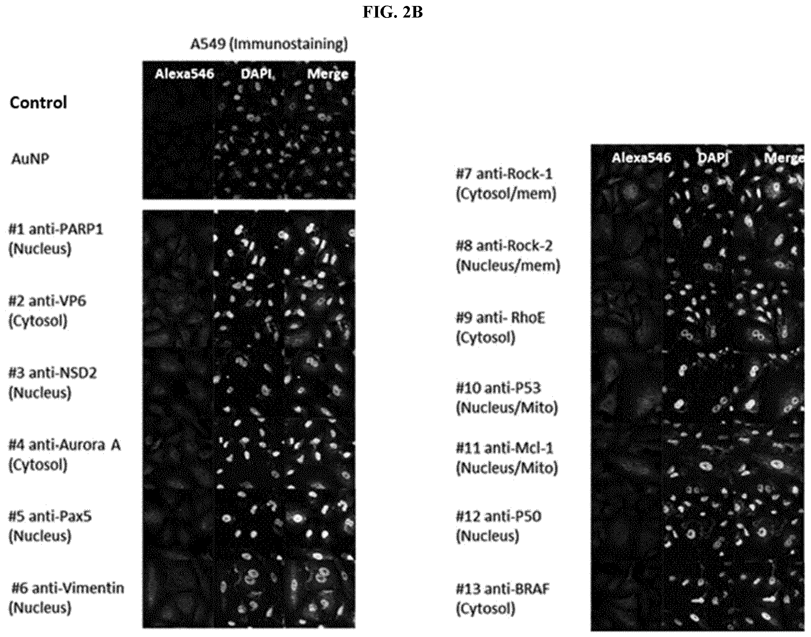

As a result, as shown in FIGS. 2A and 2B, it was confirmed that the antibodies were delivered into the HeLa (FIG. 2A) and A549 (FIG. 2B) cells.

### Example 4. Confirmation of capability of delivering antibody into cells by AuNP-Apt^{FITC}

Human skin cancer cell lines (Malme-3M and SK-MEL2) were treated with an AuNP-aptamer-antibody complex (AuNP-Apt^{FITC}-FITC-IgG) prepared by reacting the aptamer-AuNP conjugate (AuNP-Apt^{FITC}) prepared by the method described in Example 2-2 with an FITC-labeled monoclonal antibody IgG (FITC-IgG) and incubated for 1 hour, and then observed by confocal fluorescence microscopy. Here, the cells were visualized using a 40x water immersion objective.

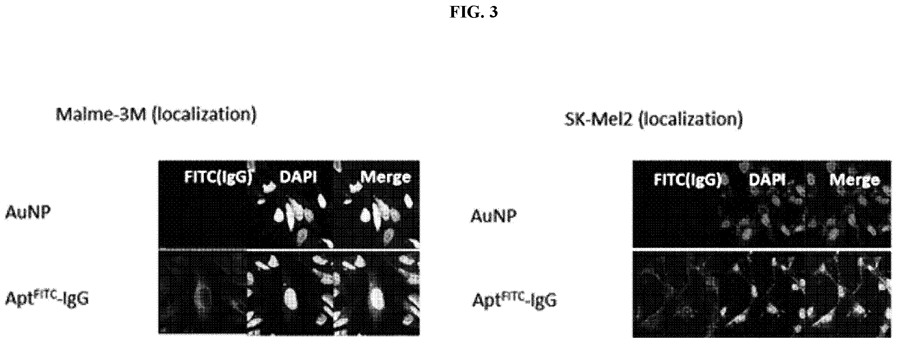

As a result, as shown in FIG. 3, it was confirmed that FITC-IgG (Green) is located in cells, demonstrating that IgG was delivered to a human skin cancer cell line.

### Example 5. Evaluation of cell viability by AuNP-aptamer-antibody complex

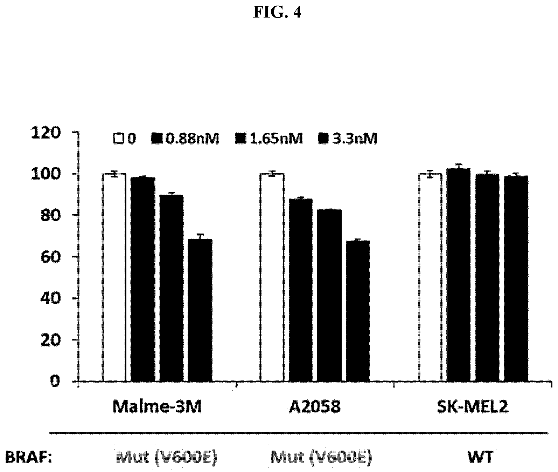

Cell viability after delivery of AuNP-Apt^{IgG}-BRAF^{V600E} into human skin cancer cell lines (A2058, SK-MEL2 and Malme-3M) was measured by the method described in Example 1-4. As a result of delivering a BRAF^{V600E} antibody used as a target molecule for treating a human skin cancer cell line into BRAF mutant cell lines A2058 and Malme-3M using AuNP-Apt^{IgG}, as shown in FIG. 4, it was confirmed that the cell viability was reduced, compared with SK-MEL2, which is a skin cancer cell line without mutation.

Therefore, from the above result, it was confirmed that the AuNP-aptamer-antibody complex can be used as a cell therapeutic agent.

### [Industrial Applicability]

An antibody carrier according to the present invention can effectively delivery an antibody into the cell nucleus, the cytoplasm and mitochondria by specific binding of the antibody to be delivered to an aptamer, and thus is expected to be effectively used to diagnose or treat a disease.

## Claims

1. An antibody carrier comprising:
a gold nanoparticle (AuNP); and
an aptamer bound to the surface of the AuNP,
wherein the aptamer consists of a base sequence of SEQ ID NO: 1 and specifically binds to the Fc domain of immunoglobulin G (IgG); or wherein the aptamer consists of a base sequence of SEQ ID NO: 2 and specifically binds to fluorescein isothiocyanate (FITC), which is a fluorescent material.

2. The antibody carrier of any one of claim 1, wherein the aptamer is bound to one or more monoclonal antibodies selected from the group consisting of an IgG antibody, PARP1 antibody, VP6 antibody, NSD2 antibody, Aurora A antibody, Pax5 antibody, Vimentin antibody, Rock-1 antibody, Rock-2 antibody, RhoE antibody, P53 antibody, Mcl-1 antibody, P50 antibody and BRAF antibody.

3. The antibody carrier of claim 1 or claim 2, wherein the AuNP has a diameter of 10 to 20 nm.

4. A method of preparing an antibody carrier, which comprises binding an aptamer to a gold nanoparticle (AuNP) wherein the aptamer consists of a base sequence of SEQ ID NO: 1 and specifically binds to the Fc domain of immunoglobulin G (IgG); or wherein the aptamer consists of a base sequence of SEQ ID NO: 2 and specifically binds to fluorescein isothiocyanate (FITC), which is a fluorescent material.

5. A complex prepared by binding the antibody carrier of claim 1 or claim 3 with an antibody to be delivered.

6. The complex of claim 5, wherein the antibody is selected from the group consisting of an IgG antibody, PARP1 antibody, VP6 antibody, NSD2 antibody, Aurora A antibody, Pax5 antibody, Vimentin antibody, Rock-1 antibody, Rock-2 antibody, RhoE antibody, P53 antibody, Mcl-1 antibody, P50 antibody and BRAF antibody.

7. A pharmaceutical composition for use in preventing or treating cancer, which comprises the complex of claim 5 as an active ingredient.

## Patentansprüche

1. Antikörperträger, umfassend:
ein Gold-Nanopartikel (AuNP); und
ein Aptamer, das an die Oberfläche des AuNP gebunden ist,
wobei das Aptamer aus einer Basensequenz von SEQ ID NO: 1 besteht und spezifisch an die Fc-Domäne von Immunglobulin G (IgG) bindet; oder wobei das Aptamer aus einer Basensequenz von SEQ ID NO: 2 besteht und spezifisch an Fluoresceinisothiocyanat (FITC) bindet, das ein fluoreszierendes Material ist.

2. Antikörperträger nach einem von Anspruch 1, wobei das Aptamer an einen oder mehrere monoklonale Antikörper gebunden ist, ausgewählt aus der Gruppe bestehend aus einem IgG-Antikörper, PARP1-Antikörper, VP6-Antikörper, NSD2-Antikörper, Aurora-A-Antikörper, Pax5-Antikörper, Vimentin-Antikörper, Rock-1-Antikörper, Rock-2-Antikörper, RhoE-Antikörper, P53-Antikörper, Mcl-1-Antikörper, P50-Antikörper und BRAF-Antikörper.

3. Antikörperträger nach Anspruch 1 oder Anspruch 2, wobei das AuNP einen Durchmesser von 10 bis 20 nm aufweist.

4. Verfahren zur Herstellung eines Antikörperträgers, das Binden eines Aptamers an ein Goldnanopartikel (AuNP) umfasst, wobei das Aptamer aus einer Basensequenz von SEQ ID NO: 1 besteht und spezifisch an die Fc-Domäne von Immunglobulin G (IgG) bindet; oder wobei das Aptamer aus einer Basensequenz von SEQ ID NO: 2 besteht und spezifisch an Fluoresceinisothiocyanat (FITC) bindet, das ein fluoreszierendes Material ist.

5. Komplex, hergestellt durch Binden des Antikörperträgers nach Anspruch 1 oder Anspruch 3 mit einem abzugebenden Antikörper.

6. Komplex nach Anspruch 5, wobei der Antikörper aus der Gruppe bestehend aus einem IgG-Antikörper, PARP1-Antikörper, VP6-Antikörper, NSD2-Antikörper, Aurora-A-Antikörper, Pax5-Antikörper, Vimentin-Antikörper, Rock-1-Antikörper, Rock-2-Antikörper, RhoE-Antikörper, P53-Antikörper, Mcl-1-Antikörper, P50-Antikörper und BRAF-Antikörper ausgewählt ist.

7. Pharmazeutische Zusammensetzung zur Verwendung bei der Vorbeugung oder Behandlung von Krebs, die den Komplex nach Anspruch 5 als Wirkstoff umfasst.

## Revendications

1. Support d'anticorps comprenant :
une nanoparticule d'or (AuNP) ; et
un aptamère lié à la surface de l'AuNP,
dans lequel l'aptamère consiste en une séquence de base de SEQ ID NO : 1 et se lie spécifiquement au domaine Fc d'une immunoglobuline G (IgG) ; ou dans lequel l'aptamère consiste en une séquence de base de SEQ ID NO : 2 et se lie spécifiquement à l'isothiocyanate de fluorescéine (FITC), qui est un matériau fluorescent.

2. Support d'anticorps selon l'une quelconque de la revendication 1, dans lequel l'aptamère est lié à un ou plusieurs anticorps monoclonaux sélectionnés dans le groupe consistant en un anticorps anti-IgG, un anticorps anti-PARP1, un anticorps anti-VP6, un anticorps anti-NSD2, un anticorps anti-aurora A, un anticorps anti-Pax5, un anticorps anti-vimentine, un anticorps anti-Rock-1, un anticorps anti-Rock-2, un anticorps anti-RhoE, un anticorps anti-P53, un anticorps anti-Mcl-1, un anticorps anti-P50 et un anticorps anti-BRAF.

3. Support d'anticorps selon la revendication 1 ou la revendication 2, dans lequel l'AuNP présente un diamètre de 10 à 20 nm.

4. Procédé de préparation d'un support d'anticorps, qui comprend l'étape consistant à lier un aptamère à une nanoparticule d'or (AuNP), dans lequel l'aptamère consiste en une séquence de base de SEQ ID NO : 1 et se lie spécifiquement au domaine Fc d'une immunoglobuline G (IgG) ; ou dans lequel l'aptamère consiste en une séquence de base de SEQ ID NO : 2 et se lie spécifiquement à l'isothiocyanate de fluorescéine (FITC), qui est un matériau fluorescent.

5. Complexe préparé en liant le support d'anticorps selon la revendication 1 ou la revendication 3 à un anticorps à délivrer.

6. Complexe selon la revendication 5, dans lequel l'anticorps est sélectionné dans le groupe consistant en un anticorps anti-IgG, un anticorps anti-PARP1, un anticorps anti-VP6, un anticorps anti-NSD2, un anticorps anti-aurora A, un anticorps anti-Pax5, un anticorps anti-vimentine, un anticorps anti-Rock-1, un anticorps anti-Rock-2, un anticorps anti-RhoE, un anticorps anti-P53, un anticorps anti-Mcl-1, un anticorps anti-P50 et un anticorps anti-BRAF.

7. Composition pharmaceutique pour une utilisation dans la prévention ou le traitement d'un cancer, qui comprend le complexe selon la revendication 5 comme principe actif.
